# EUROPEAN PATENT SPECIFICATION

(11) **EP 1 563 101 B1**
(45) Date of publication and mention of the grant of the patent: **23.04.2008**
(21) Application number: 03811765.1
(22) Date of filing: 17.11.2003
(51) Int. Cl.: C12Q 1/68

(54) **METHOD FOR THE IDENTIFICATION OF SULFO-REDUCING BACTERIA**
VERFAHREN ZUR IDENTIFIZIERUNG SULFOREDUZIERENDER BAKTERIEN
PROCEDE D'IDENTIFICATION DE BACTERIES SULFO-REDUCTRICES

(30) Priority: 22.11.2002 IT MI20022479
(43) Date of publication of application: 17.08.2005
(73) Proprietor: ENITECNOLOGIE S.p.A., 20097 S. Donato Milanese (Milano) (IT)
(72) Inventor: DE FERRA, Francesca, I-26900 Lodi (IT); LACATENA, Rosa, Margherita, I-00185 Rome (IT)
(74) Representative: De Gregori, Antonella
(86) International application number: PCT/EP2003/013043
(87) International publication number: WO 2004/048607

(56) References cited:
- GB-A- 2 354 072
- WAGNER M ET AL: "Phylogeny of dissimilatory sulfite reductase supports an early origin of sulfate respiration" JOURNAL OF BACTERIOLOGY, vol. 180, no. 11, - June 1998 (1998-06) pages 2975-82, XP002243671
- JOULIAN C ET AL: "Congruent phylogenies of most common small-subunit rRNA and dissimilatory sulfite reductase gene sequences retrieved from estuarine sediments " APPLIED AND ENVIRONMENTAL MICROBIOLOGY, vol. 67, no. 7, - July 2001 (2001-07) pages 3314-18, XP002243675 cited in the application
- MINZ D ET AL: "Diversity of sulfate-reducing bacteria in oxic and anoxic regions of a microbial mat characterized by comparative analysis of dissimilatory sulfite reductase genes" APPLIED AND ENVIRONMENTAL MICROBIOLOGY, vol. 65, no. 10, pages 4666-71, XP002243672
- LARSEN O ET AL: "A novel organization of the dissimilatory sulfite reductase operon of Thermodesulforhabdus norvegica verified by RT-PCR" FEMS MICROBIOLOGY LETTERS, vol. 203, 20 August 2001 (2001-08-20), pages 81-85, XP002243673
- THOMSEN T ET AL: "Biogeochemical and molecular signatures of anaerobic methane oxidation in a marine sediment" APPLIED AND ENVIRONMENTAL MICROBIOLOGY , vol. 67, no. 4, April 2001 (2001-04), pages 1646-56, XP002243674

## Description

The present invention relates to a method for the identification of sulfo-reducing bacteria in environmental samples.

More specifically, the present invention relates to a method for the identification of sulfo-reducing bacteria which is based on the amplification of the dissimilatory sulfite reductase (dsr) gene. Sulfate-reducing bacteria (SRB) together with sulfite and thiosulfate-reducers belong to the group of sulfo-reducing bacteria.

Sulfo-reducing bacteria are known as being the microorganisms mainly responsible for the biological formation of hydrogen sulfide (H₂S) .

This acid and, to a lesser extent, other products of the sulfo-bacteria metabolism, are the cause of numerous problems for industrial plants, in particular for those of the oil industry.

Among the most serious problems, the biological corrosion of steel and the emission into the atmosphere of harmful gases (Postgate, 1979. The sulphate reducing bacteria. Cambridge University Press, London), together with the deterioration in the quality of the oil extracted (Cord-Ruwich et al., 1987, Y. Petrol. Technol. 1: 97-106), can be mentioned.

The identification of sulfo-bacteria is consequently one of the major objectives for fighting the production of H₂S in numerous industrial activities (Tatnall et al., 1988, Material performance, 71-80).

Various methods have been developed for identifying sulfo-reducing bacteria: some of these are based on bacterial cultivation techniques (Scott & Davies, Material Performance, May 1992, 64-68), others on immunological identification techniques of APS reductase (EP 272,916), others again on tests for evaluating the hydrogenase activity, an enzyme present in sulfate-reducing bacteria (Scott and Davis, 1992).

In order to improve the sensitivity, specificity and rapidity of the sulfo-reducer identification methods, methods have recently been developed which are based on gene amplification techniques (polymerase chain reaction PCR).

These techniques have the advantage, with respect to the more commonly used bacterial culture techniques (lengthy and often unreliable), of also succeeding in identifying species not easy to cultivate, which often represent an important fraction of the bacteria present in the ecosystem.

To allow them to be used on a general scale, the techniques based on PCR must be capable of identifying the target genes only, and, preferably, all the species containing them.

The patent GB 2,354,072 describes an SRB identification method which is based on the gene amplification of at least one fragment of gene which encodes the Adenylylsulfate (APS) reductase enzyme. APS is an enzyme made up of two sub-units (α and β) and is prevalently present in most of the species of sulfate-reducing bacteria, as it is involved in the anaerobic respiration of the sulfate.

This method, however, is not capable of detecting the sulfite (and not sulfate) reducing bacteria which could be one of the causes of the formation of H₂S.

Another method for the identification of sulfo-reducing bacteria is disclosed in the Journal of Bacteriology, (1998, 180(11):2975-2982), wherein some specific reverse primer pairs are used to amplify a wide spectrum of the dissimilatory sulfate reductase (dsr) genes from different microorganisms.

Another method which is based on PCR gene amplification, is described by C. Joulian et al. (Appl. Envir. Microbiol., July 2001, 3314-3318).

The work of Joulian is oriented towards the amplification of a fragment of the gene encoding the dissimilatory sulfite reductase (dsr), an enzyme involved in the sulfate respiration process, which has at present only been found in sulfate-reducing bacteria. The sequences indicated by Joulian, however, are not general enough for the identifi-cation of many species of sulfo-reducing bacteria.

A method has now been found, based on the use of specific and "wide spectrum" primers for amplifying the dsr gene, which allows sulfo-reducing bacteria to be identified, with a high sensitivity, specificity and rapidity.

An object of the present invention therefore relates to a method for the identification of sulfo-reducing bacteria which comprises the extraction of DNA from samples in which these bacteria can be contained and the subsequent identification of at least one fragment of the gene which encodes the dsr enzyme, characterized in that the identification of the fragment of the dsr gene is effected through gene amplification in the presence of primers selected from the following pairs of oligonucleotides:

| Name | |
|---|---|
| α2F-dsr8R - | 5' TGGGAYTGGTGGATGGARGA 3' α2F |
| | 5' TAGCAGTTACCRCARTACAT 3' dsr8R |
| β1F-β1F - | 5' AACAACATHGARTTYATG 3' β1F |
| | 5' TAGCAGTTACCRCARTACAT 3' β1F |
| α1F-α2R - | 5' ACSCACTGGAAGCAGCACG 3' α1F |
| | 5' TCYTCCATCCACCARTCCCA 3' α2R |

The method of the invention has shown a greater sensitivity, specificity and rapidity with respect to the methods described in the known art (GB 2,354,072, C. Joulian) as demonstrated in the comparative tests carried out on a series of samples indicated in the examples.

The present disclosure also relates to oligonucleotides having a sequence selected among those specified above.

These oligonucleotides can not only be used as primers for gene amplification, but also as gene probes for the identification of dsr genes.

In this case, using the techniques of the known art, the disclosed oligonucleotides are subjected to labeling so that they can be easily detected, and subsequently subjected to hybridization with the genome DNA to be analyzed (as for example in the FISH technique (fluorescence in situ hybridization)) which allows specific sequences to be identified by fluorescence in samples containing cells or micro-organisms as described, for example, in "In Situ Hybridization. A practical Approach" Edited by D.G. Wilkinson IRL Press, Oxford University Press, 1994.

The labeling can be effected with different techniques such as, for example, fluorescence, radioactivity, chemiluminescence or enzymatic labeling.

The identification of the oligonucleotides of the invention has involved the use of about thirty sequences available in the NCBI data bank of the dsr gene representative of various species of Sulfate-Reducing Bacteria.

The sequences were aligned with the use of Clustal X sequence analysis software (Thomson JD et al., Nucleic Acids Research, 24:4876-4882), in order to define the preserved regions and identify, in homologous zones, the specific nucleotide sequences to be used as primers for selectively amplifying the dsr gene.

The detection method of sulfo-reducing bacteria of the invention comprises, in particular, the following passages:
- extracting the DNA from the samples;
- putting the DNA extracted in contact with a pair of primers selected from oligonucleotides having the sequences previously indicated under conditions which allow the specific amplification of a fragment of the dsr gene;
- analyzing the gene amplification product by means of real time PCR, gel-electrophoresis or with other PCR product analysis methods such as real-time PCR (Higuchi et al. 1993 Bio/Technology 11: 1026-1030).

The sample to be analyzed can consist of water coming from environmental sampling, industrial plants, bacterial cultures or fragments of biofilms on solid matrixes.

The extraction of the genome DNA from the samples to be analyzed can be effected according to the standard techniques.

As a result of the high nuclease content, the extraction is preferably carried out with cold phenol and in any case in the presence of inhibitors of the DNasic activity such as, for example, guanidinium isothiocyanate as described for example in Zinkevich and Beech (2000) Mol. Biology Today, 1: 29-33.

The use of rapid methods for the preparation of the DNA from the samples to be analyzed, such as for example the use of techniques based on rapid cellular lysis and neutralization (for example similar to those described in Kapley 2000, World J. Microbiol. Biotechnol. 16, 457-58), is also advantageous. These techniques, associated with the rapidity of the analysis with the disclosed primers, considerably reduce the detection times of sulfo-reducing bacteria, allowing them to be detected and quantified within a few hours.

The methods commonly used, on the contrary, which are based on effective bacterial cultivation capacity, require much longer times: from 1-2 days up to a week.

A pair of oligonucleotides having an identical sequence or comprising those indicated above; is used as amplification primers.

Essentially identical means that the oligonucleotide sequence is essentially identical to those previously identified or that it differs from these without influencing their capacity to hybridize with the DSR gene.

The gene amplification method used is based on the reaction of a DNA polymerase in the presence of a pair of primers and is well known to experts in the field (Sambrook et al., 1989, Molecular Cloning, Cold Spring Harbor, NY).

Conditions which allow gene amplification refer to temperature conditions, reaction times and optionally additional agents which are necessary for ensuring that the fragment of the DSR gene which hybridizes with the disclosed primers, is copied identically.

"Conditions which allow specific amplification" refer to conditions which prevent the amplification of sequences different from those of the DSR gene.

According to the method of the invention, the annealing passage during the amplification reaction is carried out at temperatures compatible with the sequence of primers, preferably, in this specific case, at 50°C.

The buffers and enzymes used are solutions compatible with the characteristics of the DNA polymerase used, such as, for example, Taq polymerase, ampliTaq Gold and polymerase hot-start, polymerase from hyperthermophile microorganisms.

Polymerases such as AmpliTaq or Taq polymerase are preferably used in the presence of the buffer solution which is most appropriate for the type of enzyme.

The sequences corresponding to the pairs of primers identified by the present invention can also be conveniently used for quantitative determinations of sulfo-reducing bacteria.

The method is based on the fact that the accumulation rate of the gene amplification product is proportional to the quantity of genome DNA present in the reaction mixture.

It is therefore possible, using instruments for quantitative PCR, to construct a calibration curve using the pairs of primers of the invention mixed with different known concentrations of genome DNA of sulfo-reducing bacteria, and fluorometrically determining the quantity of DNA produced during the amplification.

The quantity of genome DNA in the samples to be analyzed is determined by means of interpolation. The amplification can be carried out under the conditions specified by the Kits for PCR reactions, together with the conditions identified by the present invention for the use of the specific primers. The amplification is preferably carried out according to the conditions indicated in the SybrGreen™ Kit supplied by Applied Biosystems.

Screening, identification and quantization methods of sulfo-reducing bacteria such as that described by the present invention can also be advantageously exploited in bioremediation processes and environmental microbiological analysis processes in general, for example, drinking water analyses. A further advantage of the method described is the easy adaptability to protocols to be used "in situ" such as for example the use of portable instruments for real-time PCR. Another application field lies in medical diagnoses where sulfo-reducing bacteria are pathological components (for example in the field of dental medicine).

The following examples and figures illustrate the invention without limiting its scope.

### EXAMPLE 1

### Identification of specific and preserved sequences of the dsr (dissimilatory sulfite reductase) gene for selectively amplifying sulfo-reducing bacteria.

Approximately thirty sequences of the dsr gene representing different species of Sulfate-Reducing Bacteria, were recovered from the NCBI data bank. The persevered zones were sought, by means of alignment using Clustal X software, in order to design possible primers for selectively amplifying sulfo-reducing bacteria from the environmental metagenome.

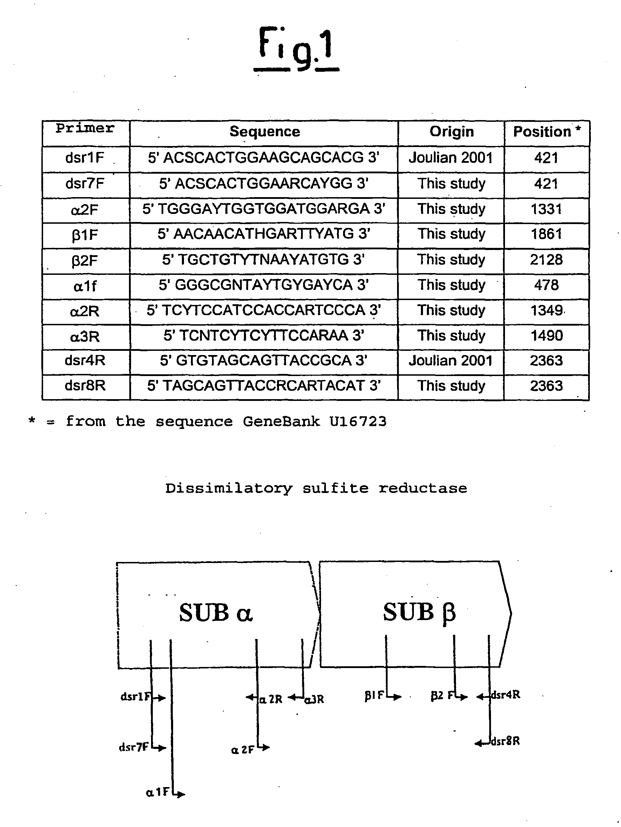

The sequences positioned as illustrated in figure 1, were thus identified. These sequences were used for the synthesis of degenerated oligonucleotides to be used as amplification primers according to what is described in the following example.

### EXAMPLE 2

### Comparison of different environmental samples of the primers dsr1F-dsr4R (known art) with the primers ET1,2,3.

The total DNA was extracted from cultures grown for 12-24 hours in Postgate medium inoculated with water from environmental samples. The cultures definitely containing SRB as indicated by the precipitation reaction of FeS in Postgate medium, were selected. The total DNA was prepared following the "rapid" protocol: 3 ml of the sample of interest are removed from the anaerobic vial with a sterile syringe and subsequently centrifuged at 14, 000 rpm and 4°C for 2 minutes; the pellet is then resuspended in 100 µl of TE. 100 µl of cold pure phenol are added and, after vortexing for a few seconds, the preparation is centrifuged at 14000 rpm and 4°C for 4 minutes and the aqueous phase is recovered. A volume of chloroform/isoamyl alcohol is added, repeating the operations of the previous step.

The aqueous phase is recovered, in which the DNA of interest is resuspended.

The volume is brought to 300 µl and the Fast DNA Kit (BIO 101) is used according to protocol, halving all the volumes of the various solutions (binding matrix, sews-M, DES) and effecting the centrifugations at 4°C. The samples of water used come from different points in the four oil centre plant, with temperatures ranging from 30°C to 50°C.

The DNA used are indicated in Table 1 below.

**Table 1**

| **Abbreviation** | **Origin** |
|---|---|
| Cv | Cavone |
| C' | Cavone |
| Fer' | Ferrandina (oil) |
| Fer | Ferrandina |
| 4 | Pisticci at 30°C |
| 34 | Pisticci at 30°C (head separator) |
| 36 | Pisticci at 30°C (head separator) |
| ST 50 | Treccate at 50°C |
| OP 50 | Treccate at 50°C |
| PG 50 | Treccate at 50°C |
| PG 30 | Treccate at 30°C |
| PG 50 | Treccate at 50°C |
| OP 50 | Treccate at 50°C |
| PG 30 | Treccate at 30°C |
| L | TO TREK |
| S | TO TREK |
| TK B | TO TREK |
| TK N | TO TREK |

The PCR reaction was carried out according to the enzyme specifications and following the conditions described in Tables 2 and 3. Following this procedure, the primers described in Example 1 were tested in all possible combinations.

The components of the reaction mixture (for a volume of 25 µl) are indicated in Table 2; the PCR program is in Table 3; the results obtained after analysis on gel-electrophoresis are summarized in Table 4.

**Table 2**

| **Reagent** | **Volume** | **Final concentration** |
|---|---|---|
| PCR buffer 10x (no Mg) | 2.5 µl | --- |
| Primer sense | 1 µl | 100 pmol./µl |
| Primer antisense | 1 µl | 100 pmol./µl |
| dNTPs (10 mM each) | 1 µl | 0.2 mM |
| DNA | 1 µl | 1-5 nq/µl |
| Taq DNA pol | 0.5 µl | 2 U |
| MgCl₂ | 1.25 µl | 1.25 Mm |
| Water | 16.75 µl | --- |

Components of the PCR reaction mixture for a volume of 250 µl.

**Table 3**

| **Number ot cycles** | **T°C** | **Time** |
|---|---|---|
| 25-30 | 94 | 50" |
| | 50 | 50" |
| | 72 | 2' |

### PCR Programs

The specific amplification reaction can obviously also be obtained with variations in the conditions indicated herein, according to techniques currently in use.

**Table 4 - Results after analysis on gel-electrophoresis of the PCR reaction product carried out on different DNA samples, using the primers described in example 1, in all possible combinations**

| **Primer F** | **Primer R** | **SRB DNA Samples** | | | | | | | | | | | | **Expected** |
|---|---|---|---|---|---|---|---|---|---|---|---|---|---|---|
| | | **CAV** | **A** | **C** | **C'** | **Pist 04** | **Pist 34** | **Pist 36** | **PG 30** | **OP 50** | **PG 50** | **ST 60** | **Ferrandina** | |
| dsr1F | α2R | -- | -- | OK | -- | | | | | | | | | 900 bp |
| dsr1F | α3R | -- | -- | -- | -- | | | | | | | | | 1010 bp |
| dsr1F | dsr4R | -- | -- | OK | | | OK | OK | | -- | -- | +/-OK | -- | 1910 bp |
| dsr1F | dsr8R | | | | | | | | | | | | | 1910 bp |
| Dsr7F | α2R | | | | | | | | | | | | | 900 bp |
| Dsr7F | α3R | | | | | | | | | | | | | 1010 bp |
| Dsr7F | dsr4R | | | | | | | | | | | | | 1910 bp |
| Dsr7F | dsr8R | OK | OK | +/-OK | OK | | | | | | | | | 1910 bp |
| α2F | dsr4R | OK | OK | OK | OK | | | | | | | | | 1060 bp |
| α2F | dsr8R | OK | OK | OK | OK | OK | OK | OK | OK | OK | OK | OK | OK | 1060 bp |
| α2F | a3R | OK | -- | OK | -- | | | | | | | | | 140 bp |
| β1F | dsr4R | +/- OK | -- | -- | OK | | | | | | | | | 480 bp |
| β1F | dsr8R | OK | OK | OK | OK | | | | OK | OK | OK | OK | | 480 bp |
| β2F | dsr4R | | -- | | -- | | | | | | | | | 220 bp |
| β2F | dsr8R | | | | | | | | | | | | | 220 bp |
| α1F | α2R | OK | OK | OK | OK | OK | OK | OK | OK | OK | OK | OK | OK | 860 bp |
| α1F | dsr4R | OK | | | -- | | | | | | | | | 1890 bp |
| α1F | α3R | -- | | | -- | | | | | | | | | 970 bp |

| | | | | | | | | | | | | | | |
|---|---|---|---|---|---|---|---|---|---|---|---|---|---|---|
| OK = expected amplification product obtained as a single band or a predominant band +/- OK = formation of a specific band but with a weak intensity or non specific bands in addition to the expected band -- = no amplification product | | | | | | | | | | | | | | |

Three pairs of primers ET1, 3 and 2 were then identified, corresponding respectively to the following sequences:

| | |
|---|---|
| ET1 α2F-dsr8R - | 5' TGGGAYTGGTGGATGGARGA 3' |
| | 5' TAGCAGTTACCRCARTACAT 3' |
| ET3 β1F-dsr8R - | 5' AACAACATHGARTTYATG 3' |
| | 5' TAGCAGTTACCRCARTACAT 3' |
| ET2 α1F-α2R - | 5' ACSCACTGGAAGCAGCACG 3' |
| | 5' TCYTCCATCCACCARTCCCA. 3' |

capable of giving positive amplification results and with a specificity which is adequate for use as diagnostic sequences for the presence of sulfo-reducing bacteria in production water. The use of some of the primers widely used in the known art, on the contrary, such as dsr1F and dsr4R is not capable of giving a positive signal on all the samples tested.

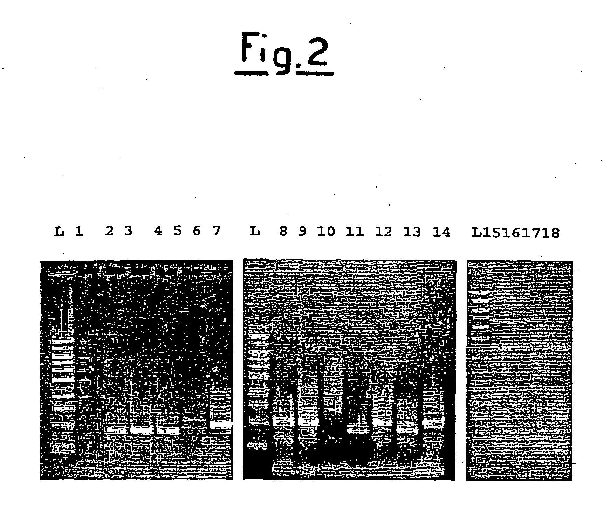

The amplification pattern of the pairs of primers identified is shown in figure 2, which indicates the analyses on gel-electrophoresis of the following samples:
L. Markers
   1. no DNA amplified with ET 2
   2. DNA 34 amplified with ET 2
   3. DNA 36 amplified with ET 2
   4. DNA F' amplified with ET 2
   5. DNA of *Rhodococcus* amplified with ET 2
   6. DNA 4 amplified with ET 1
   7. DNA 36 amplified with ET 1
   8. DNA F' amplified with ET 1
   9. DNA of *Rhodococcus* amplified with ET 1
   10. DNA Cav amplified with ET 2
   11. DNA Cav amplified with ET 1
   12. DNA C' amplified with ET 2
   13. DNA C' amplified with ET 1
   14. DNA Cav amplified with ET 3
   15. DNA *Rhodococcus* amplified with ET 3
   16. DNA *Pseudomonas* amplified with ET 3
   17. DNA ST50 amplified with ET 3

As far as *Rhodococcus* is concerned (negative blank; see figure 2), the amplifications provide poor-quality products with a completely different band dimension.

This slight non-specificity is completely eliminated if the third pair of primers, ET 3, is used, which provides a band of 480 bp and gives no amplification either with *Rhodococcus* or with *Pseudomonas.*

In conclusion, the use of the pairs of primers ET 1, Et 2 and ET 3, with respect to others previously presented, (known art) has the following advantages:
- Positive amplifications on all the samples tested, whereas dsr1F and dsr4R (Joulian 2001) do not always succeed in revealing the presence of sulfo-reducing bacteria;
- Synthesis of an amplification product with lower dimensions: 480-860-1000 bp with respect to the 1900 bp of dsr1F and dsr4R; this is a significant advantage for various reasons: the synthesis of shorter amplification products corresponds to a greater efficiency in the accumulation of the product and consequently to a greater sensitivity of the detection and quantification method.
- Our primers provide single and well distinct bands when the samples analyzed contain sulfo-reducing bacteria.
- These primers are also capable of identifying sulfo-reducing bacteria which use sulfite but not sulfate as electron acceptor, which cannot be identified with the protocols claimed in UK patent 2,354,072.

### EXAMPLE 3

### Comparison of different environmental samples of the primers APSO1 and APS11 (known art) with the primers ET1,2,3.

The primers, having the sequence 5'CGGCGCCGTTGCCCAGGG3' and 5'GGGCCGTAACCGTCCTTG3' (Ravot G. and Magot M.) claimed in UK patent 2,354,072 as specific markers for SRB, were repeatedly tested in amplification tests on the same samples from water described in Example 2, strictly following the protocol indicated in the above-mentioned patent.

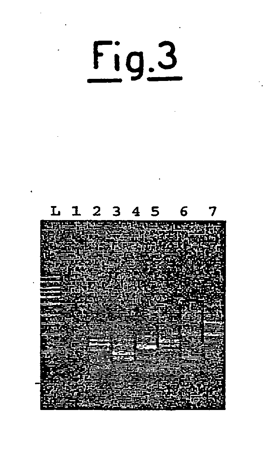

In figure 3, which indicates the analysis on gel-electrophoresis of the following samples:
L. Markers
1. Empty
2. DNA PG50 amplified with APS 01-11
3. DNA 36 amplified with APS 01-11
4. DNA Cav amplified with APS 01-11
5. DNA OP50 amplified with APS 01-11
6. DNA of *Rhodococcus* amplified with APS 01-1
7. DNA *Pseudomonas* amplified with APS 01-11
It can be observed that the above primers do not provide specific bands on mixtures of different sulfo-reducing bacteria whereas with ET 1, 2 and 3 better results are however obtained.

APS 01 and 11 are most probably not suitable for analyzing environmental samples whereas they are effective for pure cultures (such as those effectively used).

The same samples give only one amplification band with ET 1, 2 and 3 (see figure 2) .

There are some bacteria which grow directly on sulfite (and not on sulfate): in this case the enzyme which catalyzes the crucial reaction is the genic product dsr A and B, and certainly not APS reductase.

### EXAMPLE 4

### qPCR for quantifying the presence of sulfo-reducing bacteria in the processing water

The sequences corresponding to the pair ET 3 were used for quantifying the number of suite-reducing bacteria present in environmental samples.

For this purpose, a calibration curve (standard) was constructed using, both with the pair of primers ET3 and with specific primers for ribosomal DNA 16S, DNA extracted from environmental samples, definitely containing sulfate-reducing bacteria, diluted at different known concentrations.

This allows the total number of cells present in the sample to be calculated, by means of an interpolation, and, of these, the fraction representing sulfo-reducing bacteria.

The SybrGreen™ Kit supplied by Applied Biosystems was used together with the following protocol (Table 5).

**Table 5**

| **Reagent** | **Stock** | **Volume** |
|---|---|---|
| Buffer 10x SYBR green | 10 X | 2.5 µl |
| MgCl₂ | 2.5 mM | 1.25 µl (ET3) - 2.5 µl (16S) |
| DNTPs Mix | 10 mM each | 0.5 µl |
| AmpliTaq Gold or TaqPol | 5U/ul | 0.25 µl |
| Amperase | 1U/µl | 0.5 µl |
| DNA template | | 20 - 1000 pg |
| Primers | 100 pmol./ ul | 0.25 µl (16S) - 1 µl (ET3) |
| H₂O | | at 25 µl |

A Perkin Elmer 5700 instrument was used adopting the following amplification scheme in two different experiments with two types of DNA polymerase:

| - protocol for AmpliTaq Gold: | | |
|---|---|---|
| | 50°C for 2 minutes | |
| | | 1 cycle |
| | 95°C for 8 minutes | |
| | 95°C for 30 seconds | |
| | 50°C for 50 seconds | 35 cycles |
| | 60°C for 75 seconds | |

| Protocol perTaqPol: | | |
|---|---|---|
| | 95°C for 50 seconds | |
| | 50°C for 50 seconds | 30 cycles |
| | 72°C for 100 seconds | |

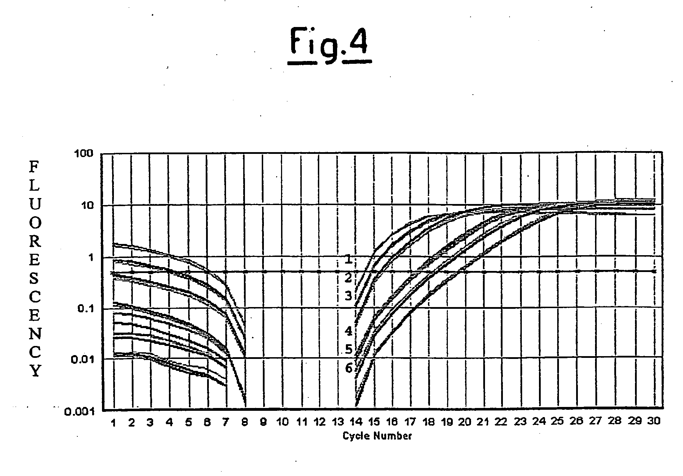

Figure 4 indicates the fluorescence curves obtained from the amplification of three different dilutions of the DNA of Cavone using universal primers for bacterial ribosomal DNA (16S rDNA) (curves 1, 2 and 3) and the primers ET3 (curves 4, 5 and 6).

It can be noted that there is a direct correlation between the concentration of the target sequence and the quantity of DNA amplified which is revealed as fluorescence for both pairs of primers. The same type of qPCR experiment was carried out with the same samples and under the same operating conditions on a portable qPCR instrument (Cepheid) with essentially identical results.

It can therefore be concluded that the primers ET3 can be used for the quantification of the target sequence present in the specific dsr gene of sulfo-reducing bacteria.

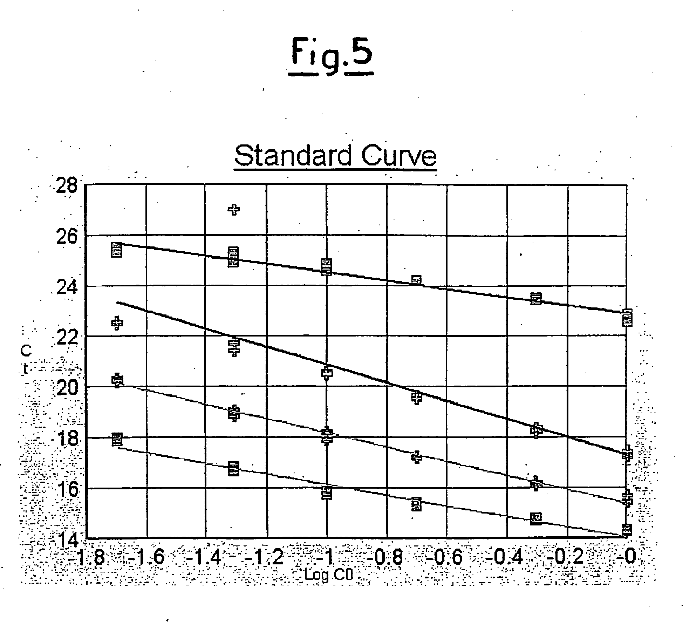

As a further confirmation of this, the test was also repeated on DNA coming from the water of Trecate and gave similar results, indicated in figure 5.

This figure shows the calibration lines for the DNA of Cavone and Trecate; said calibration curve is constructed by putting the Cₜ in ordinate and the logarithm of the initial concentrations of DNA in abscissa; subsequently, by calculating the Cₜ for the unknown samples it is possible to obtain their concentration, through an interpolation with the standard curve. The Cₜ is defined as the amplification cycle number at which the first increase in fluorescence is registered above the base line.

### Caption of figure 5:

1. Calibration curve deriving from the use of ET3 on different concentrations of DNA of micro-organisms grown from the production water of the oil centre of Trecate
2. Calibration curve deriving from the use of ET3 on different concentrations of DNA of micro-organisms grown from the production water of the oil centre of Cavone
3. Calibration curve deriving from the use of primers for 16S rDNA on different concentrations of DNA of micro-organisms grown from the production water of the oil centre of Trecate
Calibration curve deriving from the use of primers for 16S rDNA on different concentrations of DNA of micro-organisms grown from the production water of the oil centre of Cavone.

## Claims

1. A method for the identification of sulfo-reducing bacteria which comprises the extraction of DNA from samples in which these bacteria can be contained and the subsequent identification of at least one fragment of the gene which encodes the dissimilatory sulfate reductase (dsr) enzyme, **characterized in that** the identification of the fragment of the dsr gene is effected by means of gene amplification in the presence of primers selected from the pairs of oligonucleotides having the following sequences:
| name | |
|---|---|
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| α1F-α2R - | 5' ACSCACTGGAAGCAGCACG 3' |
| | 5' TCYTCCATCCACCARTCCCA 3' |

2. A pair of oligonucleotides complementary to the *dsr* gene of sulfo-reducing bacteria, selected from the following sequences:
| name | |
|---|---|
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| α1F-α2R - | 5' ACSCACTGGAAGCAGCACG 3' |
| | 5' TCYTCCATCCACCARTCCCA 3' |

3. A method for the identification of sulfo-reducing bacteria according to claim 1, comprising the following passages:
- extracting the DNA from the samples;
- putting the DNA extracted in contact with a pair of primers selected from those defined by claim 1, under conditions which allow the specific amplification of a fragment of the DSR gene;
- analyzing the gene amplification product by means of real-time PCR or gel-electrophoresis.

4. A method for the quantitative determination of sulfate-reducing bacteria, **characterized in that** the quantitative determination is effected by means of the fluorometric measurement of the gene amplification product carried out according to the method of claim 1, said method comprising:
- effecting gene amplification according to the method of claim 1, in the presence of different quantities of genome DNA of sulfate-reducing bacteria;
- the quantitative determination of the gene amplification product via fluorometry;
- the construction of a calibration curve;
- the quantitative determination of the genome DNA in the samples to be analyzed by means of interpolation.

5. The method according to claims 1 or 4, wherein the samples to be analyzed consist of water coming from oil industry plants.

## Patentansprüche

1. Verfahren zur Identifizierung von sulforeduzierenden Bakterien, welches die Extraktion von DNA aus Proben, in denen diese Bakterien enthalten sein können, sowie die nachfolgende Identifizierung wenigstens eines Fragmentes des Genes, welches das dissimilatorische Sulfatreduktase (dsr)-Enzym kodiert, umfasst, **dadurch gekennzeichnet, dass** die Identifikation des Fragmentes des dsr-Gens mittels einer Genamplifikation in der Gegenwart von Primern durchgeführt wird, welche aus den Paaren von Oligonukleotiden mit den nachfolgenden Sequenzen ausgewählt werden:
| | |
|---|---|
| Name | |
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| α1F-α2R - | 5' ACSCACTGGAAGCAGCACG 3' |
| | 5' TCYTCCATCCACCARTCCCA 3' |

2. Oligonukleotidpaar komplementär zu dem dsr-Gen von sulforeduzierenden Bakterien ausgewählt aus den nachfolgenden Sequenzen:
| Name | |
|---|---|
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| αF-α2R - | 5' ACSCACTGGAAGCAGCACG 3' |
| | 5' TCYTCCATCCACCARTCCCA 3'. |

3. Verfahren zur Identifizierung von sulforeduzierenden Bakterien nach Anspruch 1, umfassend die nachfolgenden Schritte:
- Extrahieren der DNA aus den Proben,
- Inkontaktbringen der extrahierten DNA mit einem Primerpaar, welches aus den in dem Patentanspruch 1 definierten ausgewählt wird, unter Bedingungen, welche die spezifische Amplifikation eines Fragments des DSR-Gens erlauben,
- Analysieren des Genamplifikationsprodukts mittels Real-Time-PCR oder mittels Gelelektrophorese.

4. Verfahren zur quantitativen Bestimmung von sulfatreduzierenden Bakterien, **dadurch gekennzeichnet, dass** die quantitative Bestimmung mittels fluorometrischer Messung des gemäß dem Verfahren von Patentanspruch 1 erhaltenen Genamplifikationsprodukts durchgeführt wird, wobei das Verfahren umfasst:
- Durchführen einer Genamplifikation gemäß dem Verfahren von Patentanspruch 1 in der Gegenwart von unterschiedlichen Mengen genomischer DNA von sulfatreduzierenden Bakterien,
- die quantitative Bestimmung des Genamplifikationsprodukts mittels Fluorometrie,
- Konstruktion einer Kalibrierungskurve,
- quantitative Bestimmung der genomischen DNA in den Proben, welche mittels Interpolation zu analysieren sind.

5. Verfahren nach den Ansprüchen 1 oder 4, wobei die zu analysierenden Proben aus Wasser bestehen, welches aus Ölindustrieanlagen kommt.

## Revendications

1. Procédé pour l'identification de bactéries sulfo-réductrices qui comprend l'extraction d'ADN à partir d'échantillons dans lesquels ces bactéries peuvent être contenues et l'identification ultérieure d'au moins un fragment du gène qui code l'enzyme sulfate réductase dissimulatrice (dsr), **caractérisé en ce que** l'identification du fragment du gène de la dsr est effectuée au moyen d'une amplification génique en présence d'amorces choisies parmi les paires d'oligonucléotides ayant les séquences suivantes :
| nom | |
|---|---|
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| α1f-α2R - | 5' ACSCACTGGAAGCAGCACG 3' |
| | 5' TCYTCCATCCACCARTCCCA 3' |

2. Paire d'oligonucléotides complémentaire du gène de la dsr des bactéries sulfo-réductrices, choisie parmi les séquences suivantes :
| nom | |
|---|---|
| α2F-dsr8R - | 5' TGGGAYTGGTGGATGGARGA 3' |
| | 5' TAGCAGTTACCRCARTACAT 3' |
| β1F-dsr8R - | 5' AACAACATHGARTTYATG 3' |
| | 5' TAGCAGTTACCRCARTACAT 3' |
| α1f-α2R - | 5' ACSCACTGGAAGCAGCACG 3' |
| | 5' TCYTCCATCCACCARTCCCA 3' |

3. Procédé pour l'identification de bactéries sulfo-réductrices selon la revendication 1, comprenant les passages suivants :
- l'extraction de l'ADN à partir des échantillons ;
- la mise en contact de l'ADN extrait avec une paire d'amorces choisies parmi celles définies par la revendication 1, dans des conditions qui permettent l'amplification spécifique d'un fragment du gène de la DSR ;
- l'analyse du produit d'amplification génique au moyen d'une PCR en temps réel ou d'une électrophorèse sur gel.

4. Procédé pour la détermination quantitative de bactéries réductrices des sulfates, **caractérisé en ce que** la détermination quantitative est effectuée au moyen de la mesure fluorométrique du produit d'amplification génique réalisée selon le procédé de la revendication 1, ledit procédé comprenant :
- la mise à exécution d'une amplification génique selon le procédé de la revendication 1, en présence de quantités différentes d'ADN génomique de bactéries réductrices des sulfates ;
- la détermination quantitative du produit d'amplification génique par fluorométrie ;
- la construction d'une courbe d'étalonnage ;
- la détermination quantitative de l'ADN génomique dans les échantillons à analyser au moyen d'une interpolation.

5. Procédé selon la revendication 1 ou 4, dans lequel les échantillons à analyser sont constitués d'eau provenant d'usines de l'industrie pétrolière.
